# EUROPEAN PATENT SPECIFICATION

(11) **EP 1 897 578 B1**
(45) Date of publication and mention of the grant of the patent: **14.03.2012**
(21) Application number: 05750159.5
(22) Date of filing: 16.05.2005
(51) Int. Cl.: A61M 16/20

(54) **SHUT-OFF VALVE FOR THE INTERRUPTION TECHNIQUE USED IN THE ANALYSIS OF RESPIRATORY MECHANICS**
ABSPERRVENTIL FÜR DIE UNTERBRECHUNGSTECHNIK BEI DER ANALYSE DER ATEMMECHANIK
VALVE DE FERMETURE DESTINEE A LA TECHNIQUE D'INTERRUPTION EN ANALYSE DE MECANIQUE RESPIRATOIRE

(43) Date of publication of application: 12.03.2008
(73) Proprietor: Instituto de Salud Carlos III, 28029 Madrid (ES); Fundacion Para la Investigacion Biomedica Del Hospital Universitario la Paz, 28046 Madrid (ES)
(72) Inventor: DE LA OLIVA SENOVILLA, Pedro, E-28223 POZUELO DE ALARCON (Madrid) (ES)
(74) Representative: Carpintero Lopez, Francisco
(86) International application number: PCT/ES2005/000271
(87) International publication number: WO 2006/122988

(56) References cited:
- EP-A1- 0 419 113
- WO-A1-99/47198
- ES-T3- 2 052 367
- FR-A- 1 552 128
- FR-A1- 2 659 413
- GB-A- 2 284 159
- US-B1- 6 269 813
- US-B1- 6 427 691

## Description

### OBJECT OF THE INVENTION

The present invention relates to a valve specifically conceived to cut off respiratory flow suddenly in the interruption technique, which allows measuring fundamental parameters of respiratory mechanics such as airway resistance, tissue strength and dynamic and static elasticity, referred to the respiratory system when registering tracheal pressure or the lung and thoracic cage if the oesophagus pressure is also registered.

The object of the invention is to obtain a very short closure time and a hermetic seal.

### BACKGROUND OF THE INVENTION

As mentioned above, the interruption technique allows measuring the fundamental parameters of respiratory mechanics based on recording the tracheal pressure (Pva) and oesophageal pressure (Pes), and is based on the sudden interruption of inspiratory or expiratory flow and maintaining this interruption (zero flow) for some seconds.

If the flow of the open airway is suddenly interrupted and zero flow is maintained for some seconds, the pressure vs. time curve shows three phases:
- A brusque drop in pressure if the interruption is during inspiration and brusque rise if it is during expiration. This initial brusque change in pressure is due to the resistive component of natural and/or artificial airways, such as an endotracheal tube.
- A high-frequency transitory oscillation with duration < 40 ms due to the humming of the inertive components of the respiratory system.
- A slow change in pressure until reaching equilibrium, due to the viscoelasticity and/or redistribution of gas resulting from the heterogeneity of pulmonary ventilation.

The correct determination of the start and end points of the first and third phases is essential for an accurate calculation of the respiratory mechanics parameters. This determination is difficult due to the oscillations of the second phase. An accurate determination of these points requires the valve to have two characteristics:
- A very short closure time, so that the volume that passes from the respirator to the patient (inspiratory interruption) or from the patient to the outside (expiratory interruption) is minimal;
- A hermetic seal during the time of the interruption, so that there is no loss of volume from the patient to the outside in the third phase.

These characteristics are difficult to attain, as they act in opposite senses. That is, the smaller the resistance during closing (faster occlusion) the less hermetic the occlusion.

UK patent application No. GB-2284159-A discloses an automatic resuscitator which can be held and controlled by one hand. The device can operate in three different modes - an automatic timed mode for the treatment of a patient who is not breathing which can change automatically to a demand mode when the patient commences natural inspiratory effort and a manual mode. The manual mode overrides the automatic mode to allow use of the resuscitator by a trained paramedic. The resuscitator attaches directly to a face mask using gas outlet connection and the controls are in a housing which can be held in one hand so that the other hand is left free to assist in keeping the patient's airway clear.

In the same sense, International application No. WO-99/47198-A1 relates to a transport ventilation device for administering a breathable gas under pressure to a patient by an operator, the device having controls powered by said pressurised gas in a manual override mode, a patient breathing demand override mode, and an automatic mode, the device comprising: a housing enclosing a patient breathing circuit comprising a gas accumulation chamber; a breathing chamber; breathing check valve means therebetween for permitting one way passage of inhaled gas from the accumulation chamber to the breathing chamber; and exhale check valve means for permitting passage patient exhaled breath from the breathing chamber to atmosphere external to the device; a pressure regulator having regulator input port means for admitting said gas into the device from a source of pressurised gas, and having a regulator outlet; gas distribution means for distributing gas between the regulator outlet and the gas accumulation chamber selectively via a manual circuit, a demand circuit and an automatic circuit; control means, powered solely by the pressure differential between the pressurised gas from the regulator input port means and atmospheric pressure external to the device, for monitoring and controlling said gas distribution means, wherein the control means include: breathing circuit integrity alarm means for notifying the operator that an integrity condition has been detected.

None of the above referred devices solves the problem exposed above.

### DESCRIPTION OF THE INVENTION

The shut-off valve of the invention has the two characteristics described above, allowing a clear delimitation of the aforementioned phases.

To do so and more specifically, the valve comprises two coaxial chambers, a high-pressure chamber and a low-pressure chamber, inside which move two pistons connected to each other by a common shaft, which traverses a vertical partition. The high-pressure chamber is connected to a high-pressure air supply through a servo-assisted valve that allows a near-instantaneous pressurisation of this chamber, with the resulting displacement of the shaft that moves in the low-pressure chamber. This shaft is in charge of making independent the inlet and outlet of this chamber, which are placed at a different height, in which air arrives from the respirator and is sent to the patient.

According to another characteristic of the invention, the orifice through which gas passes towards the patient has a rectangular configuration (although it may adopt other more convenient shapes such as an oval) with rounded edges and considerably elongated in a transverse direction to allow a suitable gas flow in order to minimise the maximum closure time, by making the orifice easily traversed by the piston with a short axial displacement of the latter, that is, a minimum run.

The size of the valve and the orifices of the low-pressure area must be adjusted according to the maximum gas flow that passes from the respirator to the patient through the valve, which depends on the age, weight or height and pulmonary capacity of the patient.

The valve is controlled by a computer or by a stand-alone electronic device by means of the aforementioned electrovalve that feeds the high-pressure chamber, with appropriate software and/or hardware, such that if the post-valve pressure (in the patient's airway) exceeds a predefined level the electrovalve will close the passage of pressurised air and the valve connected to the patient will spontaneously open.

### DESCRIPTION OF THE DRAWINGS

To complete the description in progress and aid a better understanding of the characteristics of the invention, according to an example of a preferred embodiment, a set of drawings is included as an integral part of the description where, for purposes of illustration and in a non-limiting sense, the following is represented:

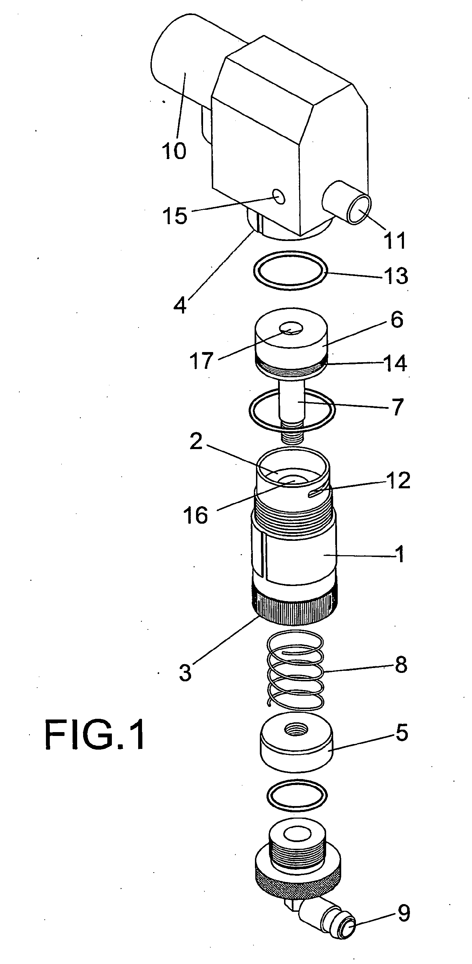

Figure 1 shows a perspective exploded view of a shut-off valve for the interruption technique used in the analysis of respiratory mechanics, executed in accordance with the object of the present invention.

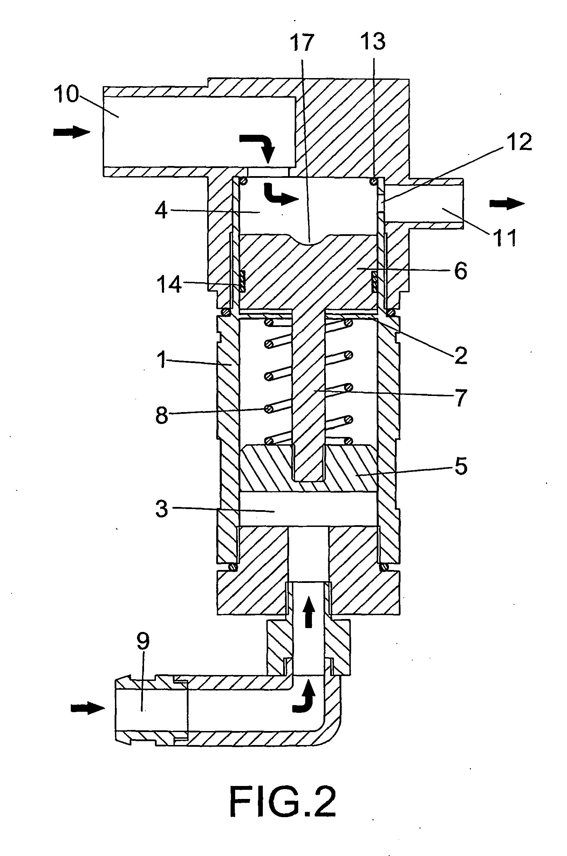

Figure 2 shows a side elevation and cross-section view of the unit of the previous figure, properly assembled.

### PREFERRED EMBODIMENT OF THE INVENTION

In view of the aforementioned drawings, it can be seen that the shut-off valve taught is based on a cylindrical valve body (1) with an intermediate partition (2) that defines inside it two coaxial chambers, a high-pressure chamber (3) and a low-pressure chamber (4), in which move the corresponding pistons (5) and (6) connected to each other by a shaft (7) that crosses the partition (2) through a central orifice (16) made in the centre of the partition (2), synchronising the movement of the two pistons.

Between the piston (5) of the high-pressure chamber (3) and the partition (2) is placed a spring (8) mounted on the shaft (7) that acts to open the valve when there is no pressure in the aforementioned high-pressure chamber (3).

The valve body (1) is closed on its lower end in correspondence with the high-pressure chamber (3), in which a gas inlet (9) is established connected to a pressure source, not shown in the drawings, through a servo-assisted electrovalve that controls the passage of gas, which in turn is governed by a computer through an analogue-digital converter or by a stand-alone electronic device, these elements not being represented in the figures.

On another hand, the low-pressure chamber (4) has an inlet (10) from the respirator and an outlet (11) to the patient. with the special characteristic that they are at a clearly different height, so that the seal is established when the piston (6) of the low-pressure chamber (4) which acts as a stopper is placed between them.

As the axial displacement of the piston (6), as stated above, is responsible for the valve opening and closing manoeuvres, to make these manoeuvres quick or nearly instantaneous the valve body (1) is provided, in correspondence with the outlet (11), with an orifice (12) in the form of a rounded rectangle, elongated in the direction perpendicular -transverse- to the shaft of the valve body (1) so that, given a proper cross-section to allow an optimum flow rate through it, the short axial distance reduces the displacement of the piston (6) when closing/opening to a minimum.

Before each use of the valve the piston (6) must be lubricated, preferably with inert silicone.

Obviously the valve is provided with sealing gaskets that ensure a proper seal between the various components.

More specifically, the low-pressure chamber (4) is hermetically sealed during occlusion by two o-rings, an upper o-ring seal (13) and a lower ring seal (14) established on the piston (6) itself that moves in said low-pressure chamber (4), the latter ring seal being a low-friction rubber x-ring seal that allows a fast sliding of two surfaces while ensuring a hermetic seal, as the sealing force increases as the system pressure increases.

To guarantee a hermetic seal, while the valve is closed the piston (6) must be in contact with the o-ring (13) and the x-ring (14) must be under the orifice (12). Therefore, the distance between the upper limit of the piston and the upper limit of the x-ring must be slightly greater than the distance between the o-ring (13) and the lower limit of the orifice (12).

In addition, to measure the pressure in the patient's airway next to the outlet (11) is a lateral orifice (15) for supplying pressure, which by the corresponding connection communicates with the cylinder that is fitted in the endotracheal tube.

The upper surface of the piston (6) has at least one depression (17) to prevent adhesion -due to the surface tension of the fluid that could fill it arriving from the humidification of the respirator circuit or the patient itself-to the roof of the low-pressure chamber (4) during closure, which would prevent or limit opening of the valve.

As can be inferred from the above, the valve as a whole is controlled by the computer or by a stand-alone electronic device using the aforementioned electrovalve that assists the high-pressure chamber (3). The resting position of this electrovalve is closed, implying that the resting position of the shut-off valve connected to the patient is open. If the electric power supply is turned off, the electrovalve closes the air flow automatically and the shut-off valve is open to allow the patient to breathe.

As described above, the size of the valve and the orifices of the low-pressure chamber must be adjusted according to the maximum gas flow from the respirator to the patient through the valve, which depends on the age, weight or height, lung capacity and respiratory pathology of the patient. The valve of the example described below has been built and tested for use in patients of paediatric age.

The time for which the valve remains closed is controlled by a computer; there are two additional safety systems, namely 1) if the post-valve pressure (in the patient's airway) exceeds a predetermined level, the electrovalve shuts off the entry of pressurised air and the valve connected to the patient opens spontaneously; and 2) the maximum time of the occlusion is limited. The computer software allows the user to define the parameters corresponding to the duration of the valve shut-off position, the post-valve safety opening pressure and the maximum valve closure time.

More specifically, the aforementioned signals used to control the electrovalve are obtained from the output of the analogue-digital converter after being processed by the computer. From the time that these control signals exit the analogue-digital converter until the valve begins to close, the time elapsed is 22 ms, which is the time required to reach the electrovalve, open it and allow the pressure to displace the valve piston. This time actually depends on the electrovalve and the computer and converter, or on the stand-alone device used, so that it will vary depending on the electrovalve and converter (or stand-alone device) chosen or designed, which properly speaking are not components of the valve object of the invention.

The displacement of the piston (6) provokes the entry of a fixed volume to the patient of 0.0876 mL (DS 0.0026) due to the compression and displacement of the gas in the low-pressure chamber (4) during shut-off. This value depends among other factors on the empty space between the piston (6) of the low-pressure chamber (4) and the upper limit of the outlet orifice (12), so that it can be reduced.

The valve closure time is estimated to be from 1 to 2 ms. This time was estimated from pressure and flow rate graphs. This can be reduced even further by shortening the resting length between the piston (6) in the low-pressure chamber (4) and the lower edge of the outlet orifice (12). The piston (6) is lubricated before each use with inert silicone, such as dimethyl-polisiloxane, as that used to lubricate endotracheal tubes.

The duration of the post-occlusion high-frequency oscillation is less than 40 ms.

The gas leak from the patient during occlusion was calculated in the valve by connecting a non-distensible closed chamber with a known volume to the distal part of the valve, that is, the part connected to the patient. Occlusion was maintained for 10 seconds at different pressures and the pressure loss was recorded. The elastance of the chamber was calculated using the air compression factor. The leak was 0.00099 mUs at 10.4 cm H₂O (DS 0.00027 mUs), 0.00220 mUs at 19.9 cm H₂O (DS 0.00020 mUs) and 0.00558 mL/s at 40.1 cm H₂O (DS 0.00012 mL/s).

The gas leak during occlusion and the shut-off time both depend on the pressure in the high-pressure chamber (3). This leak could change depending on the shut-off pressure of the piston (6) in the high-pressure chamber (3), the diameter of the piston (6) and the cross-sections of the orifice (12). The leak and shut-off time measurements for this valve have been made with a pressure of approximately 5.5 atmospheres in the high-pressure chamber (3).

The empty space in this valve was 4.7 mL. This space can be reduced by shortening the resting length between the piston (6) in the low-pressure chamber (4) and the lower edge of the outlet orifice and/or reducing the diameter of the low-pressure chamber (4), which in this example of a valve is 2cm.

## Claims

1. Shut-off valve for the interruption technique used in the analysis of respiratory mechanics, a technique that allows measuring parameters such as the airway resistance, tissue strength and dynamic and static elasticity of the respiratory system and which is based on a sudden interruption of the inspiratory or expiratory flow and maintaining this interruption for some seconds, the shut-off valve comprises a cylindrical valve body (1) in which two coaxial chambers are established, a high-pressure chamber (3) and a low-pressure chamber (4) inside which move corresponding pistons (5, 6) connected to each other by a common shaft (7), the high-pressure chamber (3) being provided with an inlet (9) for connection to a high-pressure source through a servo-assisted electrovalve, and the low-pressure chamber (4) is provided with an inlet (10) from the respirator and an outlet (11) with an orifice (12) to the patient, directly communicated in an open-valve situation and hermetically sealed by the piston (6) of the low-pressure chamber (4) when the high-pressure chamber (3) is pressurised, **characterised by** said orifice (12) having a shape that is elongated in a transverse direction, so that with a suitable cross-section for allowing an optimum rate of flow through it, the opening and closing operation taking place with a minimum axial displacement of the piston (6), wherein the high-pressure chamber (3) and low-pressure chamber (4) are established inside the valve body (1) by an intermediate partition (2) that is traversed by the shaft (7) which enters through a central orifice (16) of the intermediate partition (2) and connects the pistons (5, 6) wherein the inlet (10) and outlet (11) of the low-pressure chamber (4) are at different heights, the latter being nearer the intermediate partition (2) that separates the two chambers so that the valve shut-off is established by the interposition of the piston (6) of the low-pressure chamber between the inlet (10) and the outlet (11).

2. Shut-off valve for the interruption technique used in the analysis of respiratory mechanics, according to claim 1, **characterised in that** an upper surface of the piston (6) has at least one depression (17) that prevents, while the valve is closed, the adhesion of said upper surface of the piston (6) to the roof of the low-pressure chamber (4).

3. Shut-off valve for the interruption technique used in the analysis of respiratory mechanics, according to any of claims 1 or 2, **characterised in that** between the intermediate partition (2) that separates the high and low pressure chambers (3, 4) and the piston (5) housed inside the high-pressure chamber (3) is established a spring (8), preferably helical, that tends to displace the two pistons (5, 6) to the open-valve situation when there is no pressure in the high-pressure chamber (3).

4. Shut-off valve for the interruption technique used in the analysis of respiratory mechanics, according to any of the previous claims, **characterised in that** the orifice (12) has a rectangular shape with rounded edges.

5. Shut-off valve for the interruption technique used in the analysis of respiratory mechanics, according to any of the previous claims, **characterised in that** the distance between the upper edge of the piston (6) and an upper edge of a ring seal (14) established on the piston (6) under the orifice (12) is slightly greater than the distance between a lower edge of a ring seal (13) established in contact with the piston (6) and the lower limit of the orifice (12).

6. Shut-off valve for the interruption technique used in the analysis of respiratory mechanics, according to any of the previous claims, **characterised in that** the servo-assisted electrovalve controls the entry of gas from the high pressure source to the high-pressure chamber (3) and is controlled by a computer, this electrovalve being connected to the computer through an analogue-digital converter, or by a stand-alone electronic device.

## Patentansprüche

1. Absperrventil für die Unterbrechungstechnik bei der Analyse der Atemmechanik, einer Technik, die das Messen von Parametern wie den Atemwegwiderstand, die Gewebefestigkeit und die dynamische und statische Elastizität des Atemsystems ermöglicht und auf einer plötzlichen Unterbrechung des inspiratorischen oder des exspiratorischen Flusses und dem Beibehalten dieser Unterbrechung für einige Sekunden beruht, wobei das Absperrventil einen zylindrischen Ventilkörper (1) aufweist, in welchem zwei koaxiale Kammern ausgebildet sind, eine Hochdruckkammer (3) und eine Unterdruckkammer (4), in welchen sich jeweilige Kolben (5, 6) bewegen, die miteinander durch einen gemeinsamen Schaft (7) verbunden sind, wobei die Hochdruckkammer (3) ausgestattet ist mit einem Einlass (9) zum Verbinden mit einer Hochdruckquelle über ein vorgesteuertes Elektroventil, und wobei die Unterdruckkammer (4) mit einem respiratorseitigen Einlass (10) und einem Auslass (11) mit einer patientenseitigen Öffnung (12) versehen ist, welche bei offenem Ventil in direkter Verbindung stehen und bei druckbeaufschlagter Hochdruckkammer (3) durch den Kolben (6) der Unterdruckkammer (4) hermetisch verschlossen sind, **dadurch gekennzeichnet, dass** die Öffnung (12) eine in Querrichtung langgestreckte Form aufweist, so dass bei einem für das Ermöglichen einer optimalen Durchflussrate durch die Öffnung geeigneten Querschnitt der Öffnungs- und Schließvorgang unter minimaler axialer Verschiebung des Kolbens (6) erfolgt, wobei die Hochdruckkammer (3) und die Unterdruckkammer (4) in dem Ventilkörper (1) durch eine Zwischentrennwand (2) gebildet sind, welche von dem Schaft (7) durchquert wird, der durch eine Mittelöffnung (16) der Zwischentrennwand (2) tritt und die Kolben (5, 6) miteinander verbindet, wobei sich der Einlass (10) und der Auslass (11) der Unterdruckkammer (4) auf verschiedenen Höhen befinden, wobei der Letztere der die beiden Kammern trennenden Zwischentrennwand (2) näher ist, so dass das mittels des Ventils erfolgende Absperren durch das Anordnen des Kolbens (6) der Unterdruckkammer zwischen dem Einlass (10) und dem Auslass (11) erfolgt.

2. Absperrventil für die Unterbrechungstechnik bei der Analyse der Atemmechanik nach Anspruch 1, **dadurch gekennzeichnet, dass** eine Oberseite des Kolbens (6) mindestens eine Vertiefung (17) aufweist, welche verhindert, dass die Oberseite des Kolbens (6) im geschlossenen Zustand an der Oberwand der Unterdruckkammer (4) haftet.

3. Absperrventil für die Unterbrechungstechnik bei der Analyse der Atemmechanik nach einem der Ansprüche 1 oder 2, **dadurch gekennzeichnet, dass** zwischen der Zwischentrennwand (2), welche Hoch- und die Unterdruckkammern (3, 4) voneinander trennt, und dem in der Hochdruckkammer (3) aufgenommenen Kolben (5) eine vorzugsweise schraubenlinienförmige Feder (8) angeordnet ist, die bestrebt ist, die beiden Kolben (5, 6) in den Öffnungszustand des Ventils zu verschieben, wenn in der Hochdruckkammer (3) kein Druck herrscht.

4. Absperrventil für die Unterbrechungstechnik bei der Analyse der Atemmechanik nach einem der vorhergehenden Ansprüche, **dadurch gekennzeichnet, dass** die Öffnung (12) eine rechteckige Form mit abgerundeten Kanten aufweist.

5. Absperrventil für die Unterbrechungstechnik bei der Analyse der Atemmechanik nach einem der vorhergehenden Ansprüche, **dadurch gekennzeichnet, dass** der Abstand zwischen der oberen Kante des Kolbens (6) und der Oberkante einer an dem Kolben (6) unter der Öffnung (12) vorgesehenen Ringdichtung (14) geringfügig größer als der Abstand zwischen der Unterkante einer in Anlage an dem Kolben (6) angeordneten Ringdichtung (13) und der unteren Begrenzung der Öffnung (12) ist.

6. Absperrventil für die Unterbrechungstechnik bei der Analyse der Atemmechanik nach einem der vorhergehenden Ansprüche, **dadurch gekennzeichnet, dass** das vorgesteuerte Elektroventil das Eintreten von Gas aus der Hochdruckquelle in die Hochdruckkammer (3) regelt und durch einen Computer gesteuert ist, wobei das Elektroventil durch einen Analog-Digital-Wandler oder durch eine eigenständige elektronische Vorrichtung mit dem Computer verbunden ist.

## Revendications

1. Vanne de fermeture pour la technique d'interruption utilisée dans l'analyse de mécaniques respiratoires, une technique qui permet la mesure de paramètres comme de la résistance des voies aériennes, de la résistance du tissu et de l'élasticité dynamique et statique du système respiratoire et qui est basée sur une interruption brusque de l'écoulement inspiratoire ou expiratoire et pour maintenir cette interruption pendant quelques secondes, la vanne de fermeture comprend un corps de vanne cylindrique (1) dans lequel deux chambres coaxiales sont établies, une chambre de haute pression (3) et une chambre de basse pression (4) à l'intérieur desquelles se déplacent des pistons correspondants (5, 6) reliés l'un à l'autre par un arbre commun (7), la chambre de haute pression (3) présentant une entrée (9) pour la connection à une source de haute pression par une électrovanne servo-assistée, et la chambre de basse pression (4) présente une entrée (10) du respirateur et une sortie (11) avec un orifice (12) vers le patient, en communication directe dans une situation de vanne ouverte et hermétiquement scellée par le piston (6) de la chambre de basse pression (4) lorsque la chambre de haute pression (3) est pressurisée, **caractérisée en ce que** ledit orifice (12) a une forme qui est oblongue dans une direction transversale de sorte qu'avec une section transversale appropriée pour permettre un débit d'écoulement optimum à travers celui-ci, l'opération d'ouverture et de fermeture a lieu avec un déplacement axial minimum du piston (6), où la chambre de haute pression (3) et la chambre de basse pression (4) sont établies à l'intérieur du corps de vanne (1) par une séparation intermédiaire (2) qui est traversée par l'arbre (7) qui entre à travers un orifice central (16) de la séparation intermédiaire (2) et relie les pistons (5, 6), où l'entrée (10) et la sortie (11) de la chambre de basse pression (4) sont à des hauteurs différentes, la dernière étant plus proche de la séparation intermédiaire (2) qui sépare les deux chambres de sorte que la fermeture de vanne est établie par l'interposition du piston (6) de la chambre de basse pression entre l'entrée (10) et la sortie (11).

2. Vanne de fermeture pour la technique d'interruption utilisée dans l'analyse de mécaniques respiratoires selon la revendication 1, **caractérisée en ce qu'**une surface supérieure du piston (6) présente au moins un creux (17) qui empêche, pendant que la vanne est fermée, l'adhésion de ladite surface supérieure du piston (6) au toit de la chambre de basse pression (4).

3. Vanne de fermeture pour la technique d'interruption utilisée dans l'analyse de mécaniques respiratoires selon l'une quelconque des revendications 1 ou 2, **caractérisée en ce que**, entre la séparation intermédiaire (2) qui sépare les chambres de haute et basse pression (3, 4) et le piston (5) logé à l'intérieur de la chambre de haute pression (3) est établi un ressort (8), de préférence hélicoïdal, qui a tendance à déplacer les deux pistons (5, 6) à la situation de vanne ouverte lorsqu'il n'y a pas de pression dans la chambre de haute pression (3).

4. Vanne de fermeture pour la technique d'interruption utilisée dans l'analyse de mécanismes respiratoires selon l'une quelconque des revendications précédentes, **caractérisée en ce que** l'orifice (12) a une forme rectangulaire avec des bords arrondis.

5. Vanne de fermeture pour la technique d'interruption utilisée dans l'analyse de mécanique respiratoire selon l'une quelconque des revendications précédentes, **caractérisée en ce que** la distance entre le bord supérieur du piston (6) et un bord supérieur d'un joint annulaire (14) établi sur le piston (6) sous l'orifice (12) est légèrement plus grande que la distance entre un bord inférieur d'un joint annulaire (13) établi en contact avec le piston (6) et la limite inférieure de l'orifice (12).

6. Vanne de fermeture pour la technique d'interruption utilisée dans l'analyse de mécaniques respiratoires selon l'une quelconque des revendications précédentes, **caractérisée en ce que** l'électrovanne servo-assistée contrôle l'entrée du gaz de la source de haute pression à la chambre de haute pression (3) et est commandée par un ordinateur, cette électrovanne étant reliée à l'ordinateur par un convertisseur analogique-numérique ou par un dispositif électronique autonome.
